# EUROPÄISCHE PATENTSCHRIFT

(11) **EP 1 663 096 B1**
(45) Veröffentlichungstag und Bekanntmachung des Hinweises auf die Patenterteilung: **24.10.2007**
(21) Anmeldenummer: 04787013.4
(22) Anmeldetag: 24.09.2004
(51) Int. Cl.: A61F 13/62

(54) **VERFAHREN ZUM ANBRINGEN VON WIEDERVERSCHLÜSSEN AN BABYWINDELN**
METHOD FOR THE APPLICATION OF REUSABLE FASTENERS ON DIAPERS
PROCEDE D'APPLICATION DE FERMETURES REUTILISABLES SUR DES COUCHES DE BEBE

(30) Priorität: 25.09.2003 DE 10344536
(43) Veröffentlichungstag der Anmeldung: 07.06.2006
(73) Patentinhaber: Nordenia Deutschland Gronau GmbH, 48599 Gronau (DE)
(72) Erfinder: WILLING, Christoph, 48691 Vreden (DE)
(74) Vertreter: Albrecht, Rainer Harald
(86) Internationale Anmeldenummer: PCT/EP2004/010775
(87) Internationale Veröffentlichungsnummer: WO 2005/030104

(56) Entgegenhaltungen:
- EP-A- 0 793 953
- EP-A- 0 818 188
- US-B1- 6 471 804

## Beschreibung

Die Erfindung betrifft ein Verfahren zum Anbringen eines aus einem Verschlussband und einem Verschlussstreifen bestehenden Wiederverschlusses an einer Babywindel, wobei Streifen, die aus einem Träger und einem aufkaschierten Material mit Verschlusselementen in Form von Maschen oder Haken bestehen, an der Babywindel ohne Klebstoff befestigt werden.

Das aufkaschierte Material des Verschlussbandes bzw. Verschlussstreifens kann textiltechnisch hergestellt werden. Die zum Beispiel durch Wirken hergestellten Maschen oder Haken wirken nach Art eines Hook-and-Loop-Systems zusammen, wobei hakenförmige Verschlusselemente des einen textilen Materials in Maschen des anderen textilen Materials eingreifen. Der Träger des Verschlussbandes und/oder Verschlussstreifens kann aus einer ein- oder mehrschichtigen Folie, einem textilen Material oder einem Laminat aus einer Trägerfolie und einem ein- oder beidseitig aufkaschierten textilen Material bestehen. Ferner kann der Träger in Streifenlängsrichtung einen Bereich mit unterschiedlicher Elastizität aufweisen. Zweckmäßig sind jedoch zumindest die Anschlussbereiche des Trägers, an denen die Verbindung zu der Babywindel hergestellt werden, als dehnungsarme Bereiche ausgebildet. Als Materialien für den Träger des Verschlussbandes und Verschlussstreifens eignen sich insbesondere Polyolefine.

Im Rahmen der bekannten Maßnahmen werden die Verschlussbänder und Verschlussstreifen mittels eines Klebers, der an der Rückseite des Trägers aufgebracht wird, an der Windel befestigt. Die Befestigung erfolgt an der Windelaußenhaut oder an einer z. B. als Windelohr ausgebildeten Komponente (DE 199 40 185 A1). Die Handhabung von mit einem Kleber versehenen Materialien stellt an die Verarbeitung hohe Anforderungen. Insbesondere muss sichergestellt werden, dass die klebenden Teile bei der Verarbeitung entweder mit Releasefolien abgedeckt sind oder erst zum gewünschten Zeitpunkt mit ihrer klebenden Seite in Kontakt mit anderen Materialien kommen. Aufgrund dieser Problematik wird in DE 199 52 417 A1 vorgeschlagen, die Streifen auf der Seite der Verschlussglieder mit einer silikonhaltigen Trennschicht zu beschichten, die nach dem Beschichten ausgehärtet werden muss. Das Verfahren ist aufwendig.

Aus DE 197 03 557 A1 ist es bekannt, Verschlussstreifen durch Ultraschallverschweißen an der Außenseite einer Babywindel zu befestigen. Als weitere Möglichkeiten zur Herstellung mechanischer Bindungen kommen Verbindungen durch Wärme und/oder Druck in Betracht (EP 0 877 589 B1). Das Ultraschallverschweißen sowie das Verbinden durch Wärme und Druck sind verhältnismäßig langsame Befestigungsverfahren. Die erforderliche Verweilzeit zur Herstellung einer festen Verbindung ist deutlich länger als die für eine Kontaktklebung benötigte Zeit. Das Befestigen der einen Wiederverschluss bildenden Streifen an Babywindeln durch Wärme und Druck erscheint daher für die Praxis weniger geeignet und hat sich nicht durchsetzen können.

Aus der US 6 471 804 B1 ist bekannt, Windelteile in einem ersten Schritt zu nächst durch Ultraschallverschweißen an Materialbahnen zu heften und in einem zweiten Schritt durch Ultraschallverschweißen vollständigmiteinander zu verbinden.

Der Erfindung liegt die Aufgabe zugrunde, die Befestigung der Verschlussbänder und Verschlussstreifen an der Babywindel zu vereinfachen. Das Verfahren soll in die Windelherstellung integrierbar sein und darf die Produktionsgeschwindigkeit der Windelfertigung nicht beeinträchtigen.

Die Aufgabe wird bei dem eingangs beschriebenen Verfahren erfindungsgemäß dadurch gelöst, dass die Streifen in einem ersten Verfahrensschritt durch Thermobondieren oder Ultraschallverschweißen angeheftet und in einem zweiten Verfahrensschritt durch Kaltverpressen mit der Gegenfläche fest verbunden werden. Die beiden Verfahrensschritte werden in räumlich getrennten Arbeitsstationen durchgeführt.

Durch Thermobondieren erfolgt eine Verbindung der Materialien unter Verwendung von heißen Werkzeugen. Bei einer Ultraschallverschweißung werden hochfrequent oszillierende Stempelwerkzeuge eingesetzt, die Reibungswärme erzeugen. Durch lokales Aufschmelzen und Fließvorgänge werden die zu verbindenden Materialien im ersten Verfahrensschritt lediglich fixiert. In einem zweiten Verfahrensschritt in einer räumlich getrennten Arbeitsstation werden die Materialien anschließend durch Kaltverpressen fest verbunden. Bei einer fortlaufenden Windelproduktion können die beiden Verfahrensschritte in den separaten Arbeitsstationen zeitgleich ausgeführt werden. Durch die Aufteilung des Befestigungsverfahrens in zwei Verfahrensschritte kann die Verweildauer, die bei einer fortlaufenden Windelproduktion für das Applizieren und das Befestigen der Verschlussstreifen und Verschlussbänder benötigt wird, um bis zu 50 % reduziert werden.

Gemäß einer bevorzugten Ausführung der Erfindung werden die Streifen von einer Materialbahn mit einem Schnitt quer zur Bahnlaufrichtung abgetrennt und mittels einer Übergabevorrichtung der ersten Arbeitsstation zugeführt, in der die Streifen auf eine Bahn, aus der die Windeln oder Teile der Windeln gefertigt werden, aufgebracht und durch Thermobondieren oder Ultraschallschweißen angeheftet werden. Als Übergabevorrichtung kann insbesondere eine rotierende Übergabevorrichtung verwendet werden, welche die Streifen mit einer zur Laufrichtung der Bahn gleichsinnigen Drehbewegung der Arbeitsstation zuführt. Danach wird die Bahn durch die zweite Arbeitsstation geführt, in der die Streifen fest mit der Bahn verbunden werden.

Mit dem erfindungsgemäßen Verfahren können Verbindungen zwischen Streifen und Windeln erzeugt werden, die aus dicht nebeneinander angeordneten Befestigungspunkten bestehen.

Im Folgenden wird die Erfindung anhand einer lediglich ein Ausführungsbeispiel darstellenden Zeichnung erläutert. Es zeigen schematisch
- **Fig. 1**: ein Verfahren zum Anbringen eines aus einem Verschlussband und einem Verschlussstreifen bestehenden Wiederverschlusses an einer Babywindel,
- **Fig. 2**: das nach dem beschriebenen Verfahren hergestellte Verfahrenserzeugnis.

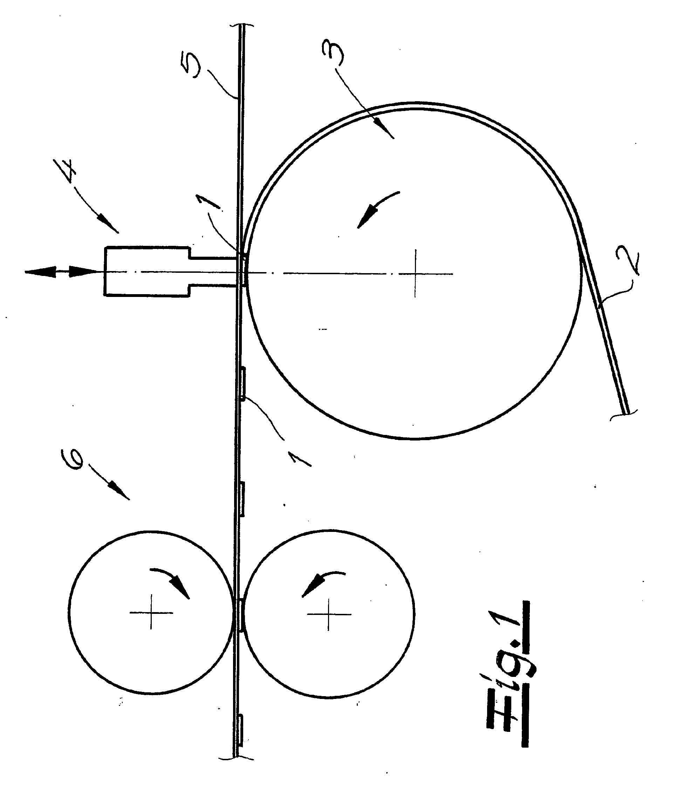

Bei dem in Fig. 1 dargestellten Verfahren werden Streifen 1 von einer Materialbahn 2 mit einem Schnitt quer zur Bahnlaufrichtung abgetrennt und mittels einer rotierenden Übergabevorrichtung 3 einer ersten Arbeitsstation 4 zugeführt. Die von der Materialbahn 2 abgetrennten Streifen 1 bestehen aus einem Träger und einem aufkaschierten Material mit Verschlusselementen in Form von Maschen oder Haken. In der ersten Arbeitsstation 4 werden sie auf eine Bahn 5, aus der Windeln oder Teile von Windeln gefertigt werden, aufgebracht und durch Thermobondieren oder Ultraschallschweißen angeheftet. Die in der ersten Arbeitsstation 4 erzeugte Verbindung dient lediglich der Fixierung der Streifen 1 an einer Fläche, welche die Außenseite der Windeln bildet. Danach wird die Bahn durch eine zweite Arbeitsstation geführt, in der die Streifen 1 fest durch Kaltverpressen mit der Bahn 5 verbunden werden.

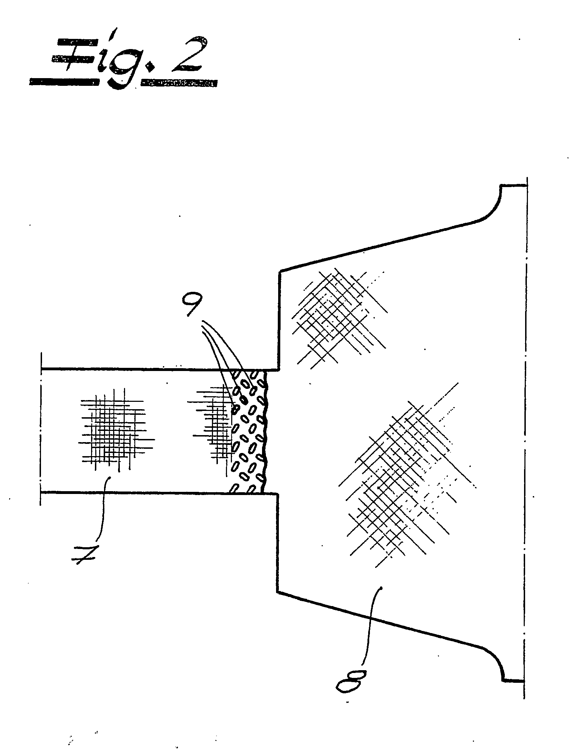

Das nach dem beschriebenen Verfahren hergestellte Verfahrenserzeugnis ist in Fig. 2 dargestellt. Die Figur 2 zeigt schematisch einen Ausschnitt aus einer Babywindel mit einem seitlichen Verschlussband 7, das an einem als Windelohr 8 ausgebildeten Anschlussbereich der Windel befestigt ist und mit einem an der Windelaußenseite angeordneten, in der Figur nicht dargestellten, Verschlussstreifen als Klettverschluss zusammenwirkt. Das Verschlussband 7 besteht aus einem Träger und einem aufkaschierten Material mit hakenförmigen Verschlusselementen, die mit maschenförmigen Verschlusselementen des an der Windelaußenseite befestigten Verschlussstreifens zusammenwirken. Das aufkaschierte Material ist textiltechnisch herstellbar.

Der Träger des Verschlussbandes kann aus einer ein- oder mehrschichtigen Folie, einem textilen Material, z. B. Nonwoven oder Gewirke, oder einem Laminat aus einer Trägerfolie und einem ein- oder beidseitig aufkaschierten textilen Material bestehen. Der Träger kann in Streifenlängsrichtung Bereiche mit unterschiedlicher Elastizität aufweisen. Vorzugsweise ist der Anschlussbereich des Trägers, der zur Befestigung des Trägers an der Babywindel benutzt wird, als dehnungsarmer Bereich ausgebildet.

Die nach dem zuvor beschriebenen Verfahren hergestellte Verbindung zwischen Streifen und Windel besteht aus dicht nebeneinander angeordneten Befestigungspunkten 9, die durch lokales Aufschmelzen und/oder Fließvorgänge unter Anwendung von Druck erzeugt worden sind.

Auch der Verschlussstreifen, der aus einem Träger und einem aufkaschierten Material mit weiblichen Verschlusselementen in Form von Maschen besteht, wird in der beschriebenen Weise an der Windelaußenseite ohne Klebstoff befestigt, wobei der Verschlussstreifen in einem ersten Verfahrensschritt durch Thermobondieren oder Ultraschallverschweißen nur angeheftet wird und in einem zweiten Verfahrensschritt durch Kaltverpressen mit der Gegenfläche dann fest verbunden wird.

## Patentansprüche

1. Verfahren zum Anbringen eines aus einem Verschlussband und einem Verschlussstreifen bestehenden Wiederverschlusses an einer Babywindel, wobei Streifen, die aus einem Träger und einem aufkaschierten Material mit Verschlusselementen in Form von Maschen oder Haken bestehen, an der Babywindel ohne Klebstoff befestigt werden, **dadurch gekennzeichnet, dass** die Streifen in einem ersten Verfahrensschritt durch Thermobondieren oder Ultraschallverschweißen angeheftet und in einem zweiten Verfahrensschritt durch Kaltverpressen mit der Gegenfläche fest verbunden werden.

2. Verfahren nach Anspruch 1, **dadurch gekennzeichnet, dass** die Streifen von einer Materialbahn mit einem Schnitt quer zur Bahnlaufrichtung abgetrennt und mittels einer Übergabevorrichtung der ersten Arbeitsstation zugeführt werden, in der die Streifen auf eine Bahn, aus der die Windeln oder Teile der Windeln gefertigt werden, aufgebracht und durch Thermobondieren oder Ultraschallverschweißen angeheftet werden, und dass die Bahn danach durch die zweite Arbeitsstation geführt wird, in der die Streifen fest mit der Bahn verbunden werden.

3. Verfahren nach Anspruch 2, **dadurch gekennzeichnet, dass** eine rotierende Übergabevorrichtung verwendet wird, welche die Streifen mit einer zur Laufrichtung der Bahn gleichsinnigen Drehbewegung der Arbeitsstation zuführt.

4. Verfahren nach einem der Ansprüche 1 bis 3, **dadurch gekennzeichnet, dass** zwischen Streifen und Windel eine Verbindung erzeugt wird, die aus dicht nebeneinander angeordneten Befestigungspunkten besteht.

## Claims

1. A method of attaching a resealable closure comprising a closure tape and a closure strip to a nappy, wherein strips consisting of a carrier and a laminated material with closure elements in the form of loops or hooks, are secured to the nappy without adhesive,
**characterised in that** the strips are affixed in a first process stage by thermobonding or ultrasound welding and are firmly connected in a second process stage by cold pressing against the opposing surface.

2. The method according to claim 1, **characterised in that** the strips are detached from a web of material by means of a cut made perpendicular to the running direction of the web and are supplied by means of a delivery device to the first work station, in which the strips are applied to a web from which the nappies or parts of the nappies are produced and affixed by thermobonding or ultrasound welding and that the web is then conducted through the second work station in which the strips are firmly connected to the web.

3. The method according to claim 2, **characterised in that** a rotating delivery device is used, which supplies the strips with a rotational movement of the work station in the same direction as the running direction of the web.

4. The method according to one of the claims 1 to 3, **characterised in that** a connection is made between the strips and nappy, which comprises attachment points disposed close to one another.

## Revendications

1. Procédé de montage d'une fermeture repositionnable consistant en une bande de fermeture et en un ruban de fermeture sur une couche pour bébé, des rubans consistant en un support et en une matière contrecollée avec des éléments de fermeture sous forme de mailles ou de crochets étant fixés sur la couche pour bébé sans utilisation de colle, **caractérisé en ce que** dans une première étape du procédé, les rubans sont attachés par thermobonding ou par soudage aux ultrasons et dans une deuxième étape du procédé, ils sont assemblés solidement avec la surface antagoniste, par compression à froid.

2. Procédé selon la revendication 1, **caractérisé en ce que** les rubans sont séparés d'un lé de matière par une coupe à la transversale du sens de déplacement du lé et ils sont acheminés au moyen d'un dispositif de transfert vers le premier poste de travail, dans lequel le ruban est appliqué sur un lé, à partir duquel on fabrique les couches ou des parties de la couche et attachés par thermobonding ou par soudage aux ultrasons et **en ce que** ledit lé est alors acheminé vers le deuxième poste de travail, dans lequel les rubans sont solidement reliés avec le lé.

3. Procédé selon la revendication 2, **caractérisé en ce qu'**on utilise un dispositif de transfert rotatif, qui achemine les rubans vers le poste de travail, par un mouvement de rotation dans le même sens que le sens de déplacement du lé.

4. Procédé selon l'une quelconque des revendications 1 à 3, **caractérisé en ce que**, entre le ruban et la couche, il est crée un assemblage, consistant dans des points de fixation disposés côte à côte de façon dense.
